# EUROPÄISCHE PATENTSCHRIFT

(11) **EP 1 480 603 B1**
(45) Veröffentlichungstag und Bekanntmachung des Hinweises auf die Patenterteilung: **24.08.2011**
(21) Anmeldenummer: 03743314.1
(22) Anmeldetag: 20.02.2003
(51) Int. Cl.: A61K 8/37, A61K 8/60, A61K 31/7024, A61Q 7/02, A61Q 17/00, A61Q 17/04, A61Q 19/00, A61Q 19/02, A61Q 19/08

(54) **VERWENDUNG VON ZUCKERESTERN IN KOSMETISCHEN ZUBEREITUNGEN**
USE OF SUGAR ESTERS IN COSMETIC PREPARATIONS
UTILISATION DES ESTERS DE SUCRES DANS DES PREPARATIONS COSMETIQUES

(30) Priorität: 01.03.2002 EP 02290508
(43) Veröffentlichungstag der Anmeldung: 01.12.2004
(73) Patentinhaber: Cognis France, S.A.S., 77310 St. Fargeau Ponthierry (FR)
(72) Erfinder: PAULY, Gilles, F-54000 Nancy (FR); FREIS, Olga, F-54280 Seichamps (FR); DANOUX, Louis, F-54420 Saulxures Les Nancy (FR); GILLON, Véronique, F-54270 Essey-les-Nancy (FR); MOUSSOU, Philippe, F-54000 Nancy (FR); GRISONI, Philippe, 54420 Saulxures les Nancy (FR)
(74) Vertreter: Gittinger, Andreas
(86) Internationale Anmeldenummer: PCT/EP2003/001731
(87) Internationale Veröffentlichungsnummer: WO 2003/074013

(56) Entgegenhaltungen:
- EP-A- 0 343 671
- EP-A- 0 375 388
- EP-A- 0 875 239
- WO-A-02/36130
- WO-A-88/06880
- DE-A- 10 019 752
- FR-A- 2 696 467
- US-A- 4 031 303
- US-A- 5 730 972
- DATABASE WPI Section Ch, Week 200152 Derwent Publications Ltd., London, GB; Class A96, AN 1996-175657 XP002211709 & JP 03 202136 B (KANEBO LTD), 27. August 2001 (2001-08-27)
- DATABASE WPI Section Ch, Week 199805 Derwent Publications Ltd., London, GB; Class B02, AN 1998-046907 XP002211710 & JP 09 295927 A (YAGI A), 18. November 1997 (1997-11-18)
- DATABASE WPI Section Ch, Week 198421 Derwent Publications Ltd., London, GB; Class D21, AN 1984-131227 XP002211711 & JP 59 067215 A (SUGA K), 16. April 1984 (1984-04-16)
- DATABASE CA [Online] CHEMICAL ABSTRACTS SERVICE, COLUMBUS, OHIO, US; MASAKI, HITOSHI ET AL: "Skin-lightening and antiaging skin preparations containing cystine esters" retrieved from STN Database accession no. 126:242616 XP002211707 & JP 09 030953 A (NOEVIR KK, JAPAN) 4. Februar 1997 (1997-02-04)
- DATABASE CA [Online] CHEMICAL ABSTRACTS SERVICE, COLUMBUS, OHIO, US; MASAKI, HITOSHI: "Antiaging cosmetic skin preparations containing 3,4,5- trihydroxybenzoic acid esters" retrieved from STN Database accession no. 123:92932 XP002211708 & JP 07 133218 A (NOEVIR KK, JAPAN) 23. Mai 1995 (1995-05-23)
- DATABASE WPI Section Ch, Week 198946 Derwent Publications Ltd., London, GB; Class A96, AN 1989-336076 XP002211981 & JP 01 250313 A (POLA CHEM IND INC), 5. Oktober 1989 (1989-10-05)
- DATABASE WPI Section Ch, Week 199618 Derwent Publications Ltd., London, GB; Class D21, AN 1996-175647 XP002237617 & JP 08 053326 A (KANEBO LTD), 27. Februar 1996 (1996-02-27)
- PATENT ABSTRACTS OF JAPAN vol. 1998, no. 01, 30. Januar 1998 (1998-01-30) & JP 09 252746 A (NIPPON KANKYO YAKUHIN KK), 30. September 1997 (1997-09-30)
- DATABASE WPI Section Ch, Week 199737 Derwent Publications Ltd., London, GB; Class A25, AN 1997-399396 XP002237618 & JP 09 175946 A (LION CORP), 8. Juli 1997 (1997-07-08)

## Beschreibung

### Gebiet der Erfindung

Die Erfindung befindet sich auf dem Gebiet der Kosmetik und betrifft die Verwendung von Zuckerestern als Wirkstoffe für die Herstellung von Haut- und Haarpflegemitteln.

### Stand der Technik

Für die Herstellung von kosmetischen Zubereitungen, speziell Hautpflegemittel werden Wirkstoffe benötigt, die sich über ein komplexes Anforderungsprofil auszeichnen : sie sollen speziell der Haut, aber auch den Haaren Schutz gegen über Umweltgiften, oxidativen Stress und UV-Strahlung verleihen, der Faltenbildung vorbeugen, anti-inflammatorisch wirksam, aber gleichzeitig auch besonders hautmild sein. Da mit der Entwicklung und Zulassung solcher "kosmetischen Vielzweckwaffen" stets ein hoher Kosten- und Zeitaufwand verbunden ist, besteht ein besonderes Interesse an solchen Wirkstoffen, die bereits für den Einsatz in Kosmetik und Pharmazie bekannt und zugelassen sind oder für die eine Herstellungsmethode beschrieben ist, deren Wirkstoffpotential bisher jedoch nicht bekannt oder nicht hinreichend erforscht ist.

Seit vielen Jahren haben nichtionische Tenside vom Typ der Glycosidfettsäureester, insbesondere Fettsäureester mit Saccharose, gemeinhin als Zuckerester bezeichnet, wegen ihrer besonderen Hautverträglichkeit, biologischen Abbaubarkeit und hohen Emulgierfähigkeit besondere Bedeutung für zahlreiche Anwendungen gefunden, wie beispielsweise die Herstellung von Emulsionen für die Kosmetik, Körperpflegemittel, Shampoos, Haarsprays, Zahnpasten, Lippstiften, Mascaras und dergleichen. Besonders umfangreich sind Herstellung und Anwendung von Fettsäureestern der Saccharose beschrieben. Ebenso finden sich Beschreibungen der Herstellung und Verwendung von Methylglucose, Fructose und Trehalose. Ihre besondere Milde, ihr Beitrag zur Feuchtigkeitsregulierung der Haut sowie ihre Verwendung zur Verringerung des Irritationspotentials von Aniontensiden oder AHA wird beispielsweise von Desai in Cosm.Toil. 105, p99-107 (1990**),** sowie in der JP 960224502**,** EP 95904334 **und** JP 93168 beschrieben.

In diesem Zusammenhang sei auf die französischen Patentanmeldung FR-A1 9211770 (L'Oreal) verwiesen, aus der die Verwendung von Fructoseoctanoat zur Wiederherstellung des Lipidfilms auf der Haut und zur Verhinderung des transepidermalen Wasserverlustes (TEWL) entfetteter Haut bekannt ist. In den Druckschriften JP-A1 03/261711 und JP 03/197414 werden Dextroseester für die Verbesserung von Weichgriff, Kämmbarkeit und Feuchtigkeit von Haaren vorgeschlagen. Aus dem japanischen Patent JP-A1 09/241404 (Lion) ist der Einsatz von Estern der Glucose mit C6-C9-Fettsäuren gegen gram-positive Bakterien und ihre Verwendung zur Herstellung von bakteriziden Zubereitungen bekannt. In den beiden Patentanmeldungen EP-A1 0875239 und EP-A1 0985408 (BDF) wird der Einsatz von Estern von Fettsäuren mit Di- oder Oligosacchariden gegen die Haftung von Mikroorganismen auf harten Oberflächen vorgeschlagen.

In der Internationalen Anmeldung WO 02/053121 werden kosmetische Hautweißungsmittel beschrieben, die Glucose 3-5 - acyl-derivate und/oder Sucrose 6 - 8 - acylderivate mit Acylgruppen aus 3 bis 6 Kohlenstoffatomen beinhalten. Unter diesen Molekülen sind die Hautweißungseigenschaften besonders für Glucose-penta-isovalerat hervorzuheben.

Die Aufgabe der vorliegenden Erfindung hat demnach darin bestanden, solche Glycosidfettsäureester zu identifizieren, die sich als Wirkstoffe zur nicht-therapeutischen, kosmetischen Verwendung zur Inhibierung der Melaninsynthese in Haar-und hentzellen eignen.

### Beschreibung der Erfindung

Gegenstand der Erfindung ist die Anspruch 1 einfügen

Überraschenderweise wurde gefunden, dass Zuckerester, die schon seit vielen Jahren als Emulgatoren für Nahrungsmittel, Kosmetika und pharmazeutische Zubereitungen bekannt sind, schon in kleinsten Konzentrationen über ein umfangreiches Profil als Wirkstoff verfügen. Der Gegenstände der Erfindung betrifft daher ihre Verwendung.

Es hat sich gezeigt, dass Zuckerester (syn. Glycosidfettsäureester) hervorragend zur Inhibierung des Haarwachstums eingesetzt werden können. Sie verringern die Zellproliferation von humanen Keratinocyten und reduzieren das Wachstum und die Entwicklung von Haarfollikeln.
Die in den letzten Jahren zunehmende Tendenz Enthaarungsmittel einzusetzen, insbesondere an Beinen, in den Achselhöhlen oder im Gesicht , macht den Einsatz von Mitteln, die die Geschwindigkeit des Haarwachstums verringern attraktiv, da auch die Intervalle einer teilweise schmerzhaften mechanischen Enthaarung somit vergrößert werden können.

Glycosidfettsäureester verbessern das Potential der Zellen gegen oxidativen Stress und Umweltgifte. Die Messung der zellschützenden (gegen Oxidation oder Schwermetalle wie Blei) Glutathion (GSH) in humanen mit UV-A Strahlung belasteten Fibroblasten zeigte eine deutliche Verbesserung des GSH-Levels nach der Behandlung mit Monosaccharid-oder Disaccharidfettsäureestern.

Die Zuckerester schützen humane Keratinocyten außerdem vor dem schädigenden Einfluss durch UV-B-Strahlung - messbar an einer Verringerung der LDH-Freisetzung und weisen eine anti-entzündliche Wirkung auf, die sich in einer Reduktion von ausgeschiedenem PGE2 ausdrückt.
In polymorph-nuklearen neutrophilen Granulocyten zeigen Monosaccharidfettsäureester eine signifikante Inhibierung des respiratory burst-effects, einer Freisetzung reaktiver Sauerstoffradikale, die an der entzündlichen Reaktion beteiligt sind.
Zusätzlich bewirken Zuckerester eine Inhibierung der Protease-Aktivität, insbesondere der Collagenase Aktivität. Es ist bekannt, dass die Aktivität der Collagenase mit zunehmender Alterung wächst. Zuckerester können daher gegen Hautalterung vorteilhaft eingesetzt werden. Auch der Einsatz gegen Zellalterung durch UV-Strahlung, oxidativen Stress oder Umweltgifte, die zu einer erhöhten Collagenaseaktivität führen, bietet sich somit an.

Zusammenfassend können Zuckerester daher vorteilhaft zum Schutz von Haut und Haarfollikeln gegen Entzündung, Sonnenbrand, Schädigungen durch Strahlung, oxidativen Stress, Umweltgifte und Hautalterung insbesondere bei empfindlicher Haut und Kopfhaut eingesetzt werden.

Lant Erfindung konnte gezeigt werden, dass Zuckerester die Melanin Synthese in B16 Melanocyten verringern, so dass die Anwendung als Hautweißungsmittel auf der Hand liegt.

Die Anwesenheit von Propionibacterium acnes und Staphylococcus epidermidis führen zu den bekannten Hautveränderungen bei Akne. Insbesondere Propionibacterium acnes bewirkt eine verstärkte Komedonenbldung und unterstützt entzündliche Reaktionen. Es wurde nun gefunden, dass Glycosidfettsäureester nicht nur gegen Staphylococcus epidermidis wirksam sind, sondern auch gram-negative Bakterien wie Propionibacterium acnes unterdrücken und auf diesem Wege zur anti-entzündlichen Wirkung bei Akne erheblich beitragen.

### Zuckerester

Zuckerester stellen bekannte nichtionische Tenside dar, die nach den einschlägigen Methoden der präparativen organischen Chemie erhältlich sind, beispielsweise durch Umesterung von Fettsäuremethylestern mit den entsprechenden Zuckern oder auf enzymatischem Wege, wie dies beispielsweise in der internationalen Patentanmeldung WO 99/02722 (Laboratoires Sérobiologiques) beschrieben wird. Zuckerester mit unterschiedlichsten Glycosid- und Acylkomponenten sind im Handel erhältlich, beispielsweise von den Firmen Sisterna oder Ryoto. Typische Beispiele für geeignete Zuckerester sind in der folgenden Abbildung dargestellt: 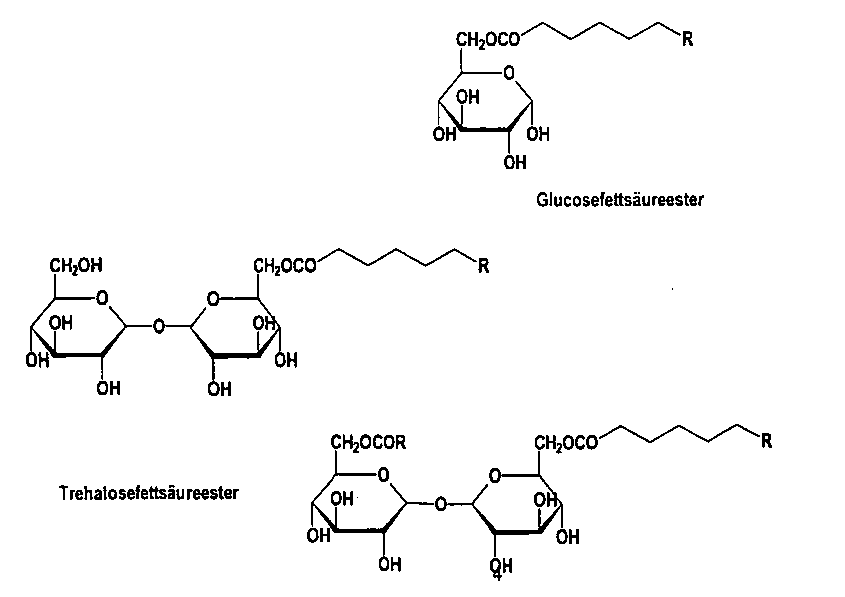 Grundsätzlich kommen Zuckerester in Frage, die sich von Mono-oder Disacchariden ableiten. Dabei kann es sich um Aldohexosen (z.B. Glucose, Methylglucose, Mannose, Galactose); Deoxyaldosen (z.B. Rhamnose, Fucose, Deoxyribose); Aldopentosen (z.B. Ribose, Arabinose, Xylose); Ketosen (z.B. Fructose in Pyranosyl- oder Furanosylform); oder Disaccha ride (z.B. Trehalose, Sucrose, Maltose, Isomaltose, Cellobiose, Melibiose, Gentobiose, Lactose) handeln. Vorzugsweise kommen Fructose-Glucose-, Trehalose- und/oder Sucroseester zum Einsatz, von diesen werden insbesondere Fructoseester bevorzugt. Die Acylkomponente der Ester kann sich von Fettsäuren der Formel **(I)** ableiten,

**R¹CO-OH** **(I)**

in der R¹CO für einen linearen oder verzweigten, gesättigten oder ungesättigten Acyl- oder Hydroxyacylest mit 6 bis 22, vorzugsweise 8 bis 16 Kohlenstoffatomen und 0 und/oder 1 bis 3 Doppelbindungen steht. Typische Beispiele sind Zuckerester der Capronsäure, 2-Hydroxycapronsäure, 6-Hydroxycapronsäure, Caprylsäure, 2-Ethylhexansäure, Caprinsäure, 10-Hydroxycaprinsäure, Laurinsäure, 12-Hydroxylaurinsäure, Isotridecansäure, Myristinsäure, Palmitinsäure, Palmoleinsäure, 16-Hydroxypalmitinsäure, Stearinsäure, Isostearinsäure, Ölsäure, Elaidinsäure, Petroselinsäure, Linolsäure, Linolensäure, Elaeostearinsäure, 12-Hydroxystearinsäure, Ricinolsäure, Arachinsäure, Gadoleinsäure, Behensäure und Erucasäure sowie deren technische Mischungen. Die Ester können je nach zur Verfügung stehender Hydroxylgruppen 1 bis 8 Estergruppen aufweisen; vorzugsweise werden jedoch solche Produkte eingesetzt, die einen Veresterungsgrad von durchschnittlich 1 bis 6 und insbesondere 1,5 bis 2,5 aufweisen.

### Kosmetische und/oder pharmazeutische Zubereitungen

Die Zuckerester werden erfindungsgemäß zur Herstellung von kosmetischen und/oder pharmazeutischen Zubereitungen wie beispielsweise Haarshampoos, Haarlotionen, Schaumbäder, Duschbäder, Cremes, Gele, Lotionen, alkoholische und wässrig/alkoholische Lösungen, Emulsionen, Wachs/ Fett-Massen, Stiftpräparaten, Pudern oder Salben verwendet, in denen sie in Mengen von 0,0001 bis 10, vorzugsweise 0,001 bis 5 und insbesondere 0,01 bis 1 Gew.-% - bezogen auf die Mittel - enthalten sind. Diese Mittel können ferner als weitere Hilfs- und Zusatzstoffe milde Tenside, Ölkörper, Emulgatoren, Perlglanzwachse, Konsistenzgeber, Verdickungsmittel, Überfettungsmittel, Stabilisatoren, Polymere, Siliconverbindungen, Fette, Wachse, Lecithine, Phospholipide, biogene Wirkstoffe, UV-Lichtschutzfaktoren, Antioxidantien, Deodorantien, Antitranspirantien, Antischuppenmittel, Filmbildner, Quellmittel, Insektenrepellentien, Selbstbräuner, Tyrosininhibitoren (Depigmentierungsmittel), Hydrotrope, Solubilisatoren, Konservierungsmittel, Parfümöle, Farbstoffe und dergleichen enthalten.

Da Zuckerester selber emulgierende, oberflächenaktive und feuchtigkeitsspendende Eigenschaften aufweisen, können sie jedoch auch teilweise ohne Zusätze weiterer oberflächenaktiver Tenside oder Emulgatoren eingesetzt werden.

### Tenside

Als oberflächenaktive Stoffe können anionische, nichtionische, kationische und/oder amphotere bzw. zwitterionische Tenside enthalten sein, deren Anteil an den Mitteln üblicherweise bei etwa 1 bis 70, vorzugsweise 5 bis 50 und insbesondere 10 bis 30 Gew.-% beträgt. Typische Beispiele für anionische Tenside sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Fettsäureethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Typische Beispiele für nichtionische Tenside sind Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, gegebenenfalls partiell oxidierte Alk(en)yloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen. Typische Beispiele für kationische Tenside sind quartäre Ammoniumverbindungen, wie beispielsweise das Dimethyldistearylammoniumchlorid, und Esterquats, insbesondere quaternierte Fettsäuretrialkanolaminestersalze. Typische Beispiele für amphotere bzw. zwitterionische Tenside sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Typische Beispiele für besonders geeignete milde, d.h. besonders hautverträgliche Tenside sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, α-Olefinsulfonate, Ethercarbonsäuren, Alkyloligoglucoside, Fettsäureglucamide, Alkylamidobetaine, Amphoacetale und/oder Proteinfettsäurekondensate, letztere vorzugsweise auf Basis von Weizenproteinen.

### Ölkörper

Als Ölkörper kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen oder verzweigten C₆-C₂₂-Fettalkoholen bzw. Ester von verzweigten C₆-C₁₃-Carbonsäuren mit linearen oder verzweigten C₆-C₂₂-Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, I-sostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von linearen C₆-C₂₂-Fettsäuren mit verzweigten Alkoholen, insbesondere 2-Ethylhexanol, Ester von C₁₈-C₃₈-Alkylhydroxycarbonsäuren mit linearen oder verzweigten C₆-C₂₂-Fettalkoholen, insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, wie z.B. Dicaprylyl Carbonate (Cetiol® CC), Guerbetcarbonate auf Basis von Fettalkoholen mit 6 bis 18, vorzugsweise 8 bis 10 C Atomen, Ester der Benzoesäure mit linearen und/oder verzweigten C₆-C₂₂-Alkoholen (z.B. Finsolv® TN), lineare oder verzweigte, symmetrische oder unsymmetrische Dialkylether mit 6 bis 22 Kohlenstoffatomen pro Alkylgruppe, wie z.B. Dicaprylyl Ether (Cetiol® OE), Ringöffnungsprodukte von epoxidierten Fettsäureestern mit Polyolen, Siliconöle (Cyclomethicone, Siliciummethicontypen u.a.) und/oder aliphatische bzw. naphthenische Kohlenwasserstoffe, wie z.B. wie Squalan, Squalen oder Dialkylcyclohexane in Betracht.

### Emulgatoren

Als Emulgatoren kommen beispielsweise nichtionogene Tenside aus mindestens einer der folgenden Gruppen in Frage:
➢ Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe sowie Alkylamine mit 8 bis 22 Kohlenstoffatomen im Alkylrest;
➢ Alkyl- und/oder Alkenyloligoglykoside mit 8 bis 22 Kohlenstoffatomen im Alk(en)ylrest und deren ethoxylierte Analoga;
➢ Anlagerungsprodukte von 1 bis 15 Mol Ethylenoxid an Ricinusöl und/oder gehärtetes Ricinusöl;
➢ Anlagerungsprodukte von 15 bis 60 Mol Ethylenoxid an Ricinusöl und/oder gehärtetes Ricinusöl;
➢ Partialester von Glycerin und/oder Sorbitan mit ungesättigten, linearen oder gesättigten, verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
➢ Partialester von Polyglycerin (durchschnittlicher Eigenkondensationsgrad 2 bis 8), Polyethylenglycol (Molekulargewicht 400 bis 5000), Trimethylolpropan, Pentaerythrit, Zuckeralkoholen (z.B. Sorbit), Alkylglucosiden (z.B. Methylglucosid, Butylglucosid, Laurylglucosid) sowie Polyglucosiden (z.B. Cellulose) mit gesättigten und/oder ungesättigten, linearen oder verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
➢ Mischester aus Pentaerythrit, Fettsäuren, Citronensäure und Fettalkohol und/oder Mischester von Fettsäuren mit 6 bis 22 Kohlenstoffatomen, Methylglucose und Polyolen, vorzugsweise Glycerin oder Polyglycerin.
➢ Mono-, Di- und Trialkylphosphate sowie Mono-, Di- und/oder Tri-PEG-alkylphosphate und deren Salze;
➢ Wollwachsalkohole;
➢ Polysiloxan-Polyalkyl-Polyether-Copolymere bzw. entsprechende Derivate;
➢ Block-Copolymere z.B. Polyethylenglycol-30 Dipolyhydroxystearate;
➢ Polymeremulgatoren, z.B. Pemulen-Typen (TR-1,TR-2) von Goodrich;
➢ Polyalkylenglycole sowie
➢ Glycerincarbonat.

### ➢ Ethylenoxidanlagerungsprodukte

Die Anlagerungsprodukte von Ethylenoxid und/oder von Propylenoxid an Fettalkohole, Fettsäuren, Alkylphenole oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind als Rückfettungsmittel für kosmetische Zubereitungen bekannt.

### ➢ Alkyl- und/oder Alkenyloligoglykoside

Alkyl- und/oder Alkenyloligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 Kohlenstoffatomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

### ➢ Partialglyceride

Typische Beispiele für geeignete Partialglyceride sind Hydroxystearinsäuremonoglycerid, Hydroxystearinsäurediglycerid, Isostearinsäuremonoglycerid, Isostearinsäurediglycerid, Ölsäuremonoglycerid, Ölsäurediglycerid, Ricinolsäuremoglycerid, Ricinolsäurediglycerid, Linolsäuremonoglycerid, Linolsäurediglycerid, Linolensäuremonoglycerid, Linolensäurediglycerid, Erucasäuremonoglycerid, Erucasäurediglycerid, Weinsäuremonoglycerid, Weinsäurediglycerid, Citronensäuremonoglycerid, Citronendiglycerid, Äpfelsäuremonoglycerid, Äpfelsäurediglycerid sowie deren technische Gemische, die untergeordnet aus dem Herstellungsprozeß noch geringe Mengen an Triglycerid enthalten können. Ebenfalls geeignet sind Anlagerungsprodukte von 1 bis 30, vorzugsweise 5 bis 10 Mol Ethylenoxid an die genannten Partialglyceride.

### ➢ Sorbitanester

Als Sorbitanester kommen Sorbitanmonoisostearat, Sorbitansesquiisostearat, Sorbitandiisostearat, Sorbitantriisostearat, Sorbitanmonooleat, Sorbitansesquioleat, Sorbitandioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitandihydroxystearat, Sorbitantrihydroxystearat, Sorbitanmonotartrat, Sorbitansesquitartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitandimaleat, Sorbitantrimaleat sowie deren technische Gemische. Ebenfalls geeignet sind Anlagerungsprodukte von 1 bis 30, vorzugsweise 5 bis 10 Mol Ethylenoxid an die genannten Sorbitanester.

### ➢ Polyglycerinester

Typische Beispiele für geeignete Polyglycerinester sind Polyglyceryl-2 Dipolyhydroxystearate (Dehymuls® PGPH), Polyglycerin-3-Diisostearate (Lameform® TGI), Polyglyceryl-4 Isostearate (Isolan® GI 34), Polyglyceryl-3 Oleate, Diisostearoyl Polyglyceryl-3 Diisostearate (Isolan® PDI), Polyglyceryl-3 Methylglucose Distearate (Tego Care® 450), Polyglyceryl-3 Beeswax (Cera Bellina®), Polyglyceryl-4 Caprate (Polyglycerol Caprate T2010/90), Polyglyceryl-3 Cetyl Ether (Chimexane® NL), Polyglyceryl-3 Distearate (Cremophor® GS 32) und Polyglyceryl Polyricinoleate (Admul® WOL 1403) Polyglyceryl Dimerate Isostearate sowie deren Gemische. Beispiele für weitere geeignete Polyolester sind die gegebenenfalls mit 1 bis 30 Mol Ethylenoxid umgesetzten Mono-, Di- und Triester von Trimethylolpropan oder Pentaerythrit mit Laurinsäure, Kokosfettsäure, Talgfettsäure, Palmitinsäure, Stearinsäure, Ölsäure, Behensäure und dergleichen.

### ➢ Anionische Emulgatoren

Typische anionische Emulgatoren sind aliphatische Fettsäuren mit 12 bis 22 Kohlenstoffatomen, wie beispielsweise Palmitinsäure, Stearinsäure oder Behensäure, sowie Dicarbonsäuren mit 12 bis 22 Kohlenstoffatomen, wie beispielsweise Azelainsäure oder Sebacinsäure.

### ➢ Amphotere und kationische Emulgatoren

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylatund eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethyl-ammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropion-säuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe.. Besonders bevorzugte ampholytische Tenside sind das N-Kokosalkylaminopropionat, das Kokosacylaminoethylaminopropionat und das C_{12/18}-Acylsarcosin. Schließlich kommen auch Kationtenside als Emulgatoren in Betracht, wobei solche vom Typ der Esterquats, vorzugsweise methylquaternierte Difettsäuretriethanolaminester-Salze, besonders bevorzugt sind.

### Fette und Wachse

Typische Beispiele für Fette sind Glyceride, d.h. feste oder flüssige pflanzliche oder tierische Produkte, die im wesentlichen aus gemischten Glycerinestern höherer Fettsäuren bestehen, als Wachse kommen u.a. natürliche Wachse, wie z.B. Candelillawachs, Carnaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z.B. Montanesterwachse, Sasolwachse, hydrierte Jojobawachse sowie synthetische Wachse, wie z.B. Polyalkylenwachse und Polyethylenglycolwachse in Frage. Neben den Fetten kommen als Zusatzstoffe auch fettähnliche Substanzen, wie Lecithine und Phospholipide in Frage. Unter der Bezeichnung Lecithine versteht der Fachmann diejenigen Glycero-Phospholipide, die sich aus Fettsäuren, Glycerin, Phosphorsäure und Cholin durch Veresterung bilden. Lecithine werden in der Fachwelt daher auch häufig als Phosphatidylcholine (PC). Als Beispiele für natürliche Lecithine seien die Kephaline genannt, die auch als Phosphatidsäuren bezeichnet werden und Derivate der 1,2-Diacyl-sn-glycerin-3-phosphorsäuren darstellen. Dem gegenüber versteht man unter Phospholipiden gewöhnlich Mono- und vorzugsweise Diester der Phosphorsäure mit Glycerin (Glycerinphosphate), die allgemein zu den Fetten gerechnet werden. Daneben kommen auch Sphingosine bzw. Sphingolipide in Frage.

### Perlglanzwachse

Als Perlglanzwachse kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxysubstituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

### Konsistenzgeber und Verdickungsmittel

Als Konsistenzgeber kommen in erster Linie Fettalkohole oder Hydroxyfettalkohole mit 12 bis 22 und vorzugsweise 16 bis 18 Kohlenstoffatomen und daneben Partialglyceride, Fettsäuren oder Hydroxyfettsäuren in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete Verdickungsmittel sind beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethyl- und Hydroxypropylcellulose, ferner höhermolekulare Polyethylenglycolmono- und -diester von Fettsäuren, Polyacrylate, (z.B. Carbopole® und Pemulen-Typen von Goodrich; Synthalene® von Sigma; Keltrol-Typen von Kelco; Sepigel-Typen von Seppic; Salcare-Typen von Allied Colloids), Polyacrylamide, Polymere, Polyvinylalkohol und Polyvinylpyrrolidon. Als besonders wirkungsvoll haben sich auch Bentonite, wie z.B. Bentone® Gel VS-5PC (Rheox) erwiesen, bei dem es sich um eine Mischung aus Cyclopentasiloxan, Disteardimonium Hectorit und Propylencarbonat handelt. Weiter in Frage kommen Tenside, wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

### Überfettungsmittel

Als Überfettungsmittel können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

### Stabilisatoren

Als Stabilisatoren können Metallsalze von Fettsäuren, wie z.B. Magnesium-, Aluminium-und/oder Zinkstearat bzw. -ricinoleat eingesetzt werden.

### Polymere

Geeignete kationische Polymere sind beispielsweise kationische Cellulosederivate, wie z.B. eine quaternierte Hydroxyethylcellulose, die unter der Bezeichnung Polymer JR 400® von Amerchol erhältlich ist, kationische Stärke, Copolymere von Diallylammoniumsalzen und Acrylamiden, quaternierte Vinylpyrrolidon/Vinylimidazol-Polymere, wie z.B. Luviquat® (BASF), Kondensationsprodukte von Polyglycolen und Aminen, quaternierte Kollagenpolypeptide, wie beispielsweise Lauryldimonium Hydroxypropyl Hydrolyzed Collagen (Lamequat®L/Grünau), quaternierte Weizenpolypeptide, Polyethylenimin, kationische Siliconpolymere, wie z.B. Amodimethicone, Copolymere der Adipinsäure und Dimethylaminohydroxypropyldiethylentriamin (Cartaretine®/Sandoz), Copolymere der Acrylsäure mit Dimethyl-diallylammoniumchlorid (Merquat® 550/Chemviron), Polyaminopolyamide, sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar® C-17, Jaguar® C-16 der Firma Celanese, quaternierte Ammoniumsalz-Polymere, wie z.B. Mirapol® A-15, Mirapol® AD-1, Mirapol® AZ-1 der Firma Miranol.

Als anionische, zwitterionische, amphotere und nichtionische Polymere kommen beispielsweise Vinylacetat/Crotonsäure-Copolymere, Vinylpyrrolidon/Vinylacrylat-Copolymere, Vinylacetat/Butylmaleat/ Isobornylacrylat-Copolymere, Methylvinylether/Maleinsäureanhydrid-Copolymere und deren Ester, unvernetzte und mit Polyolen vernetzte Polyacrylsäuren, Acrylamidopropyltrimethylammoniumchlorid/ Acrylat-Copolymere, Octylacrylamid/Methylmeth-acrylat/tert.Butylaminoethylmethacrylat/2-Hydroxypropylmethacrylat-Copolymere, Polyvinylpyrrolidon, Vinylpyrrolidon/Vinylacetat-Copolymere, Vinylpyrrolidon/ Dimethylaminoethylmethacrylat/Vinylcaprolactam-Terpolymere sowie gegebenenfalls derivatisierte Celluloseether und Silicone in Frage.

### Siliconverbindungen

Geeignete Siliconverbindungen sind beispielsweise Dimethylpolysiloxane, Methylphenylpolysiloxane, cyclische Silicone sowie amino-, fettsäure-, alkohol-, polyether-, epoxy-, fluor-, glykosid- und/oder alkylmodifizierte Siliconverbindungen, die bei Raumtemperatur sowohl flüssig als auch harzförmig vorliegen können. Weiterhin geeignet sind Simethicone, bei denen es sich um Mischungen aus Dimethiconen mit einer durchschnittlichen Kettenlänge von 200 bis 300 Dimethylsiloxan-Einheiten und hydrierten Silicaten handelt.

### UV-Lichtschutzfilter und Antioxidantien

Unter UV-Lichtschutzfaktoren sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z.B. Wärme wieder abzugeben. UVB-Filter können öllöslich oder wasserlöslich sein. Als öllösliche Substanzen sind z.B. zu nennen:
➢ 3-Benzylidencampher bzw. 3-Benzylidennorcampher und dessen Derivate, z.B. 3-(4-Methylbenzyliden)campher;
➢ 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoesäureamylester;
➢ Ester der Zimtsäure, vorzugsweise 4-Methoxyzimtsäure-2-ethylhexylester, 4-Methoxyzimtsäurepropylester, 4-Methoxyzimtsäureisoamylester 2-Cyano-3,3-phenylzimtsäure-2-ethylhexylester (Octocrylene);
➢ Ester der Salicylsäure, vorzugsweise Salicylsäure-2-ethylhexylester, Salicylsäure-4-isopropylbenzylester, Salicylsäurehomomenthylester;
➢ Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
➢ Ester der Benzalmalonsäure, vorzugsweise 4-Methoxybenzmalonsäuredi-2-ethylhexylester;
➢ Triazinderivate, wie z.B. 2,4,6-Trianilino-(p-carbo-2'ethyl-1'-hexyloxy)-1,3,5-triazin und Octyl Triazon, oder Dioctyl Butamido Triazone (Uvasorb® HEB);
➢ Propan-1,3-dione, wie z.B. 1-(4-tert.Butylphenyl)-3-(4'methoxyphenyl)propan-1,3-dion;
➢ Ketotricyclo(5.2.1.0)decan-Derivate.

Als wasserlösliche Substanzen kommen in Frage:
➢ 2-Phenylbenzimidazol-5-sulfonsäure und deren Alkali-, Erdalkali-, Ammonium-, Alkylammonium-, Alkanolammonium- und Glucammoniumsalze;
➢ Sulfonsäurederivate von Benzophenonen, vorzugsweise 2-Hydroxy-4-methoxybenzophenon-5-sulfonsäure und ihre Salze;
➢ Sulfonsäurederivate des 3-Benzylidencamphers, wie z.B. 4-(2-Oxo-3-bornylidenmethyl)benzolsulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol® 1789), 1-Phenyl-3-(4'-isopropylphenyl)-propan-1,3-dion sowie Enaminverbindungen. Die UV-A und UV-B-Filter können selbstverständlich auch in Mischungen eingesetzt werden. Besonders günstige Kombinationen bestehen aus den Derivate des Benzoylmethans" z.B. 4-tert.-Butyl-4'-methoxydibenzoylmethan (Parsol® 1789) und 2-Cyano-3,3-phenylzimtsäure-2-ethyl-hexylester (Octocrylene) in Kombination mit Ester der Zimtsäure, vorzugsweise 4-Methoxyzimtsäure-2-ethylhexylester und/oder 4-Methoxyzimtsäurepropylester und/oder 4-Methoxyzimtsäureisoamylester. Vorteilhaft werden deartige Kombinationen mit wasserlöslichen Filtern wie z.B. 2-Phenylbenzimidazol-5-sulfonsäure und deren Alkali-, Erdalkali-, Ammonium-, Alkylammonium-, Alkanolammonium-und Glucammoniumsalze kombiniert.

Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und dekorative Kosmetik verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T2000 (Merck). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt. Vorzugsweise wird mikronisiertes Zinkoxid verwendet.

Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, ZnSO₄) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

### Biogene Wirkstoffe

Unter biogenen Wirkstoffen sind beispielsweise Tocopherol, Tocopherolacetat, Tocopherolpalmitat, Ascorbinsäure, (Desoxy)Ribonucleinsäure und deren Fragmentierungsprodukte, β-Glucane, Retinol, Bisabolol, Allantoin, Phytantriol, Panthenol, AHA-Säuren, Aminosäuren, Ceramide, Pseudoceramide, essentielle Öle, Pflanzenextrakte, wie z.B. Prunusextrakt, Bambaranussextrakt und Vitaminkomplexe zu verstehen.

### Deodorantien und keimhemmende Mittel

Kosmetische Deodorantien (Desodorantien) wirken Körpergerüchen entgegen, überdecken oder beseitigen sie. Körpergerüche entstehen durch die Einwirkung von Hautbakterien auf apokrinen Schweiß, wobei unangenehm riechende Abbauprodukte gebildet werden. Dementsprechend enthalten Deodorantien Wirkstoffe, die als keimhemmende Mittel, Enzyminhibitoren, Geruchsabsorber oder Geruchsüberdecker fungieren.

### ➢ Keimhemmende Mittel

Als keimhemmende Mittel sind grundsätzlich alle gegen grampositive Bakterien wirksamen Stoffe geeignet, wie z. B. 4-Hydroxybenzoesäure und ihre Salze und Ester, N-(4-Chlorphenyl)-N' -(3,4 dichlorphenyl)harnstoff, 2,4,4'-Trichlor-2'-hydroxy-diphenytether (Triclosan), 4-Chlor-3,5-dimethyl-phenol, 2,2'-Methylen-bis(6-brom-4-chlorphenol), 3-Methyl-4-(1-methylethyl)-phenol, 2-Benzyl-4-chlorphenol, 3-(4-Chlorphenoxy)-1,2-propandiol, 3-Iod-2-propinylbutylcarbamat, Chlorhexidin, 3,4,4'-Trichlorcarbanilid (TTC), antibakterielle Riechstoffe, Thymol, Thymianöl, Eugenol, Nelkenöl, Menthol, Minzöl, Farnesol, Phenoxyethanol, Glycerinmonocaprinat, Glycerinmonocaprylat, Glycerinmonolaurat (GML), Diglycerinmonocaprinat (DMC), Salicylsäure-N-alkylamide wie z. B. Salicylsäure-n-octylamid oder Salicylsäure-n-decylamid.

### ➢ Enzyminhibitoren

Als Enzyminhibitoren sind beispielsweise Esteraseinhibitoren geeignet. Hierbei handelt es sich vorzugsweise um Trialkylcitrate wie Trimethylcitrat, Tripropylcitrat, Triisopropylcitrat, Tributylcitrat und insbesondere Triethylcitrat (Hydagen® CAT). Die Stoffe inhibieren die Enzymaktivität und reduzieren dadurch die Geruchsbildung. Weitere Stoffe, die als Esteraseinhibitoren in Betracht kommen, sind Sterolsulfate oder -phosphate, wie beispielsweise Lanosterin-, Cholesterin-, Campesterin-, Stigmasterin- und Sitosterinsulfat bzw -phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbonsäuren und deren Ester wie beispielsweise Citronensäure, Äpfelsäure, Weinsäure oder Weinsäurediethylester, sowie Zinkglycinat.

### ➢ Geruchsabsorber

Als Geruchsabsorber eignen sich Stoffe, die geruchsbildende Verbindungen aufnehmen und weitgehend festhalten können. Sie senken den Partialdruck der einzelnen Komponenten und verringern so auch ihre Ausbreitungsgeschwindigkeit. Wichtig ist, daß dabei Parfums unbeeinträchtigt bleiben müssen. Geruchsabsorber haben keine Wirksamkeit gegen Bakterien. Sie enthalten beispielsweise als Hauptbestandteil ein komplexes Zinksalz der Ricinolsäure oder spezielle, weitgehend geruchsneutrale Duftstoffe, die dem Fachmann als "Fixateure" bekannt sind, wie z. B. Extrakte von Labdanum bzw. Styrax oder bestimmte Abietinsäurederivate. Als Geruchsüberdecker fungieren Riechstoffe oder Parfümöle, die zusätzlich zu ihrer Funktion als Geruchsüberdecker den Deodorantien ihre jeweilige Duftnote verleihen. Als Parfümöle seien beispielsweise genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen sowie Harzen und Balsamen. Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, p-tert.-Butylcyclohexylacetat, Linalylacetat, Phenylethylacetat, Linalylbenzoat, Benzylformiat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labdanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilat, Irotyl und Floramat allein oder in Mischungen, eingesetzt.

### ➢ Antitranspirantien

Antitranspirantien (Antiperspirantien) reduzieren durch Beeinflussung der Aktivität der ekkrinen Schweißdrüsen die Schweißbildung, und wirken somit Achselnässe und Körpergeruch entgegen. Wässrige oder wasserfreie Formulierungen von Antitranspirantien enthalten typischerweise folgende Inhaltsstoffe:
➢ adstringierende Wirkstoffe,
➢ Ölkomponenten,
➢ nichtionische Emulgatoren,
➢ Coemulgatoren,
➢ Konsistenzgeber,
➢ Hilfsstoffe wie z. B. Verdicker oder Komplexierungsmittel und/oder
➢ nichtwässrige Lösungsmittel wie z. B. Ethanol, Propylenglykol und/oder Glycerin.

Als adstringierende Antitranspirant-Wirkstoffe eignen sich vor allem Salze des Aluminiums, Zirkoniums oder des Zinks. Solche geeigneten antihydrotisch wirksamen Wirkstoffe sind z.B. Aluminiumchlorid, Aluminiumchlorhydrat, Aluminiumdichlorhydrat, Aluminiumsesquichlorhydrat und deren Komplexverbindungen z. B. mit Propylenglycol-1,2. Aluminiumhydroxyallantoinat, Aluminiumchloridtartrat, Aluminium-Zirkonium-Trichlorohydrat, Aluminium-Zirko-nium-tetrachlorohydrat, Aluminium-Zirkonium-pentachlorohydrat und deren Komplexverbindungen z. B. mit Aminosäuren wie Glycin. Daneben können in Antitranspirantien übliche öllösliche und wasserlösliche Hilfsmittel in geringeren Mengen enthalten sein. Solche öllöslichen Hilfsmittel können z.B. sein:
➢ entzündungshemmende, hautschützende oder wohlriechende ätherische Öle,
➢ synthetische hautschützende Wirkstoffe und/oder
➢ öllösliche Parfümöle.

Übliche wasserlösliche Zusätze sind z.B. Konservierungsmittel, wasserlösliche Duftstoffe, pH-Wert-Stellmittel, z.B. Puffergemische, wasserlösliche Verdickungsmittel, z.B. wasserlösliche natürliche oder synthetische Polymere wie z.B. Xanthan-Gum, Hydroxyethylcellulose, Polyvinylpyrrolidon oder hochmolekulare Polyethylenoxide.

### Filmbildner

Gebräuchliche Filmbildner sind beispielsweise Chitosan, mikrokristallines Chitosan, quaterniertes Chitosan, Polyvinylpyrrolidon, Vinylpyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen.

### Antischuppenwirkstoffe

Als Antischuppenwirkstoffe kommen Pirocton Olamin (1-Hydroxy-4-methyl-6-(2,4,4-trimythylpentyl)-2-(1H)-pyridinonmonoethanolaminsalz), Baypival® (Climbazole), Ketoconazol®, (4-Acetyl-1-{-4-[2-(2.4-dichlorphenyl) r-2-(1H-imidazol-1-ylmethyl)-1,3-dioxylan-c-4-ylmethoxyphenyl}piperazin, Ketoconazol, Elubiol, Selendisulfid, Schwefel kolloidal, Schwefelpolyehtylenglykolsorbitanmonooleat, Schwefelrizinolpolyehtoxylat, Schwfel-teer Destillate, Salicylsäure (bzw. in Kombination mit Hexachlorophen), Undexylensäure Monoethanolamid Sulfosuccinat Na-Salz, Lamepon® UD (Protein-Undecylensäurekondensat), Zinkpyrithion, Aluminiumpyrithion und Magnesiumpyrithion / Dipyrithion-Magnesiumsulfat in Frage.

### Quellmittel

Als Quellmittel für wäßrige Phasen können Montmorillonite, Clay Mineralstoffe, Pemulen sowie alkylmodifizierte Carbopoltypen (Goodrich) dienen.

### Insekten-Repellentien

Als Insekten-Repellentien kommen N,N-Diethyl-m-toluamid, 1,2-Pentandiol oder Ethyl Butylacetylaminopropionate in Frage

### Selbstbräuner und Depigmentierungsmittel

Als Selbstbräuner eignet sich Dihydroxyaceton. Als Tyrosinhinbitoren, die die Bildung von Melanin verhindern und Anwendung in Depigmentierungsmitteln finden, kommen beispielsweise Arbutin, Ferulasäure, Kojisäure, Cumarinsäure und Ascorbinsäure (Vitamin C) in Frage.

### Hydrotrope

Zur Verbesserung des Fließverhaltens können ferner Hydrotrope, wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind
➢ Glycerin;
➢ Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
➢ technische Oligoglyceringemische mit einem Eigenkondensationsgrad von 1,5 bis 10 wie etwa technische Diglyceringemische mit einem Diglyceringehalt von 40 bis 50 Gew.-%;
➢ Methyolverbindungen, wie insbesondere Trimethylolethan, Trimethylolpropan, Trimethylolbutan, Pentaerythrit und Dipentaerythrit;
➢ Niedrigalkylglucoside, insbesondere solche mit 1 bis 8 Kohlenstoffen im Alkylrest, wie beispielsweise Methyl- und Butylglucosid;
➢ Zuckeralkohole mit 5 bis 12 Kohlenstoffatomen, wie beispielsweise Sorbit oder Mannit,
➢ Zucker mit 5 bis 12 Kohlenstoffatomen, wie beispielsweise Glucose oder Saccharose;
➢ Aminozucker, wie beispielsweise Glucamin;
➢ Dialkoholamine, wie Diethanolamin oder 2-Amino-1,3-propandiol.

### Konservierungsmittel

Als Konservierungsmittel eignen sich beispielsweise Phenoxyethanol, Formaldehydlösung, Parabene, Pentandiol oder Sorbinsäure sowie die unter der Bezeichnung Surfacine® bekannten Silberkomplexe und die in Anlage 6, Teil A und B der Kosmetikverordnung aufgeführten weiteren Stoffklassen.

### Parfümöle und Aromen

Als Parfümöle seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, α-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilllat, Irotyl und Floramat allein oder in Mischungen, eingesetzt.

Als Aromen kommen beispielsweise Pfefferminzöl, Krauseminzöl, Anisöl, Sternanisöl, Kümmelöl, Eukalyptusöl, Fenchelöl, Citronenöl, Wintergrünöl, Nelkenöl, Menthol und dergleichen in Frage.

### Farbstoffe

Als Farbstoffe können die für kosmetische Zwecke geeigneten und zugelassenen Substanzen verwendet werden. Beispiele sind Kochenillerot A (C.I. 16255), Patentblau V (C.I.42051), Indigotin (C.1.73015), Chlorophyllin (C.I.75810), Chinolingelb (C.I.47005), Titandioxid (C.I.77891), Indanthrenblau RS (C.I. 69800) und Krapplack (C.I.58000). Als Lumineszenzfarbstoff kann auch Luminol enthalten sein. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% - bezogen auf die Mittel - betragen. Die Herstellung der Mittel kann durch übliche Kalt - oder Heißprozesse erfolgen; vorzugsweise arbeitet man nach der Phaseninversionstemperatur-Methode.

### Beispiele

In den nachfolgenden Beispielen wurden enzymatisch hergestellte Zuckerester der Fructose, Glucose und Trehalose eingesetzt, welche gemäß der Vorschrift der Internationalen Patentanmeldung WO 99/02722 (Laboratoire Serobiologiques) hergestellt worden waren. Die Reinigung erfolgte durch Flüssig/Flüssig-Extraktion oder Extraktion mit überkritischem Kohlendioxid. Bei den eingesetzten Saccharoseestern (SE) handelte es sich um Verkaufsprodukte der Firmen Sisterna und Ryoto. Die genaue Zusammensetzung der Ester ist in Tabelle 1 wiedergegeben:

**Tabelle 1:**

| **Zusammensetzung der eingesetzten Zuckerester** | | | |
|---|---|---|---|
| **Zuckerester** | **Mono/Diester-Verhältnis** | **Restgehalt Zucker Gew.-%** | **Restgehalt Fettsäure Gew.-%** |
| Fructosecaprat | 58 :42 | 5 | < 5 |
| Fructosedicaprat | 9:91 | < 3 | < 5 |
| Fructosepalmitat | 49 : 51 | < 3 | < 5 |
| Fructosestearat | 49 : 51 | < 3 | < 3 |
| Fructosemonostearat | > 98 : < 2 | < 3 | < 3 |
| Glucosecaprylat | > 98 : < 2 | < 5 | 11 |
| Glucoselaurat | > 98 : < 2 | < 5 | 8 |
| Glucosepalmitat | > 98 : < 2 | < 5 | < 5 |
| Trehalosecaprat | 57 : 43 | < 5 | < 5 |
| Trehaloselaurat | 33 : 77 | < 5 | < 5 |
| Trehalosepalmitat | 52 : 48 | < 5 | < 5 |
| Trehalosestearat | 48 : 52 | < 5 | < 5 |

### A) Regenerative Wirkung und Entgiftungspotential (Illustratives Beispiel)

Glutathion (GSH) ist ein spezielles Protein, welches von den Zellen zum Schutz gegen oxidativen Stress und Umweltgifte, insbesondere gegen Schwermetalle produziert wird. Die an der reduzierten Form des GSH beteiligten drei Aminosäuren sind mit speziellen cytoplasmatischen Enzymen verknüpft, die zur Aktivierung ATP benötigen. Ein Anstieg der GSH-Konzentration führt zu einem Anstieg der Glutathion-S-transferase-Aktivität, eines Entgiftungsenzyms. Die Stimulation zur Entgiftung durch die Testsubstanzen wurden an menschlichen Fibroblasten durch Messung des GSH untersucht. Zu diesem Zweck wurden in einer Testreihe Fibroblasten zunächst 3 Tage bei 37 °C in einer Nährlösung und dann 3 Tage bei gleicher Temperatur in einer Testlösung inkubiert. Anschließend wurde der Proteingehalt in den Zellen nach der Bradford-Methode sowie die GSH-Konzentration nach der Hissin-Methode [siehe Analytical Biochem. 74, 214-226, 1977] bestimmt. Die Ergebnisse sind in Tabelle 2 zusammengefasst. Angege-ben sind die Ergebnisse von jeweils 3 Messreihen mit Dreifachbestimmung in %-rel gegen-über einer Blindprobe.

**Tabelle 2**

| **Wachstums- und überlebensstimulierende Wirkung (Angabe in %-rel.)** | | |
|---|---|---|
| **Zuckerester** | **Konz. %w/v** | GSH/proteine |
| Blindprobe | 0 | 100 |
| Trehalosepalmitat | 0,001 | 125 |
| Trehalosestearat | 0,001 | 141 |

Die Beispiele zeigen, dass die Testsubstanzen den Metabolismus im Hinblick auf das Wachstum und den Schutz in Form des Entgiftungspotentials der Fibroblasten stimulieren.

### B) Anti-stress Wirkung gegenüber UV-A-geschädigten Fibroblasten (Illustratives Beispiel)

UV-A-Strahlung im Bereich von 320 bis 400 nm induziert einen oxidativen Stress der hauptsächlich durch die Aktivierung von photosensitiven biologischen Komponenten hervorgerufen wird, welche ihrerseits die Bildung von ROS-ähnlichen Superoxidanionen, Wasserstoffperoxid und Singulettsauerstoffatomen katalysieren. Die Anti-UV-A-Wirkung wurde an Fibroblasten untersucht, da die UV-A-Strahlung durch die Dermis tritt, wo sie eine oxidative Schädigung durch Lipoperoxidation der Zellmembran sowie einer Abnahme des Gehaltes an reduzierten Glutathions (GHS) bewirkt. In diesem Sinne wurden menschliche Fibroblasten wie unter A) bereits beschrieben kultiviert, mit UV-A-Strahlen (20 J/Cm2) behandelt und dann die Zellzahlen sowie der GSH-Gehalt bestimmt. Die Ergebnisse sind in Tabelle 3 zusammengefasst. Angegeben sind die Ergebnisse von jeweils 3 Messreihen mit Dreifachbestimmung in %-rel gegenüber einer Blindprobe.

**Tabelle 3**

| **Anti-Stresswirkung (Angabe in %-rel.)** | | | |
|---|---|---|---|
| **Zuckerester** | **Konz. % w/v** | **Zellproteine** | **GSH/Proteine** |
| Blindprobe | 0 | 100 | 100 |
| Blindprobe + UV-A | 0 | 105 | 65 |
| Fructosepalmitat + UV-A | 0,003 | 91 | 81 |
| Fructosestearat + UV-A | 0,003 | 83 | 79 |
| Glucosecaprat + UV-A | 0,003 | 109 | 144 |
| Glucoselaurat + UV-A | 0,0001 | 95 | 91 |
| Glucosepalmitat + UV-A | 0,003 | 119 | 114 |
| Trehalosecaprat + UV-A | 0,00003 | 120 | 87 |
| Trehaloselaurat + UV-A | 0,0003 | 119 | 90 |
| Trehalosepalmitat + UV-A | 0,001 | 124 | 87 |
| Trehalosestearat + UV-A | 0,001 | 124 | 84 |

Durch den Einsatz der Zuckerester wurde der Gehalt an GSH in den Fibroblasten gegenüber der UV-A-Einstrahlung geschützt.

### C) Zellschutz gegenüber UV-B-Strahlen (Illustratives Beispiel)

Aufgabe dieses Tests war es zu zeigen, dass die Testsubstanzen anti-inflammatorische Eigenschaften gegenüber menschlichen Keratinocyten besitzen. UVB wurde als Stressfaktor ausgewählt, weil die Strahlen durch Aktivierung von Arachidonsäure freisetzenden Enzymen, wie beispielsweise Phospholipase A2 (PLA2) eine cutane Inflammation (Erytheme, Ödeme) hervorrufen. Dies führt in der Folge nicht nur zu einer Schädigung der Membranen, sondern auch zur Bildung von inflammatorisch wirkenden Stoffen, wie beispielsweise Prostaglandinen vom Typ PGE2. Der Einfluss der UVB-Strahlen auf die Keratinocyten wurde in-vitro über die Freisetzung von cytoplasmatischen Enzymen, wie z.B. LDH (Lactat Dehydrogenase) bestimmt, die parallel zur Zellschädigung und der Bildung von PGE2 verläuft. Zur Versuchsdurchführung wurde eine Fibroblastenkultur mit fötalem Kalbsserum angesetzt und 2 Tage später mit den Testsubstanzen geimpft. Nach einer Inkubation von 36 h bei 37 °C und einem CO₂-Level von 5 Vol.-% wurde das Nährmedium durch eine Elektrolytlösung ersetzt und die Fibroblasten mit einer definierten UVB-Strahlungsmenge geschädigt (50 mJ/cm²). Die Keratinocytenmenge wurde nach Trypsination über einen Zellzähler ermittelt, die LDH-Konzentration enzymatisch bestimmt. Die Ergebnisse sind in Tabelle 4 zusammengefasst. Angegeben ist die Aktivität in %-rel gegen einen Standard als Mittelwert von zwei Testreihen mit Doppelbestimmung.

**Tabelle 4**

| **Wirkung gegen UVB-Strahlen (Angabe in %-rel.)** | | | | |
|---|---|---|---|---|
| **Zuckerester** | **Konz. %:w/v** | **Cellulare DNA** | **Freigesetztes LDH** | **Freigesetztes PGE2** |
| Blindprobe | 0 | 100 | 0 | 0 |
| Blindprobe + UV-B | 0 | 33 | 100 | 100 |
| Fructosecaprat + UV-B | 0,001 | 32 | 68 | 35 |
| | 0,003 | 140 | 0 | 0 |
| Glucosecaprat + UV-B | 0,003 | 28 | 72 | 50 |
| | 0,01 | 36 | 48 | 28 |
| Glucoselaurat + UV-B | 0,001 | 17 | 105 | 107 |
| | 0,003 | 54 | 49 | 23 |
| Glucosepalmitat + UV-B | 0,001 | 32 | 68 | 63 |
| | 0,003 | 32 | 68 | 65 |

Die Ergebnisse zeigen, dass die Testsubstanzen die schädigenden Einflüsse von UVB-Strahlen signifikant reduzieren und insbesondere die Freisetzung von LDH und PGE2 vermindern.

### D) Anti-inflammatorische Wirkung (Illustratives Beispiel)

Im Verlauf einer cutanen Inflammation werden Leucocyten, wie beispielsweise die polymorphonuclearen neutrophilen Granulocyten (PMN) durch Peptide wie etwa Cytokine stimuliert, Botschafterstoffe wie z.B. Leukotrien auszusenden, die von aktivierten oder nekrotischen Zellen in der Dermis freigesetzt werden. Diese aktivierte PMN setzen nicht nur proinflammatorische Cytokine, Leukotriene und Proteasen, sondern auch ROS, wie z.B. Superoxide und Hypochloritanionen frei, welche die Aufgabe haben, eingedrungene pathogene Keime oder Pilze zu vernichten. Diese Aktivität der PMN während der Inflammation ist als sogenannter Atmungsausbruch ("respiratory burst") bekannt. Zur Untersuchung, inwiefern die Testsubstanzen den Atmungsausbruch verhindern oder mindern können, wurde eine Zelllinie von menschlichen leukemischen Granulocyten dieser PMN zusammen mit den Testsubstanzen bei 37 °C von 5 Vol.-% CO₂ inkubiert. Nach Auslösung des Atmungsausbruchs durch Zugabe eines Hefeextraktes (Zymosan) zur Zelllösung, wurde die Freisetzung von Superoxidanionen über deren Reaktion mit Luminol bestimmt. Die Ergebnisse sind in Tabelle 5 zusammengefasst. Angegeben sind die Zellzahlen sowie die Menge an freigesetzten ROS in %-rel zum Standard als Mittelwert einer Messreihe mit Dreifachbestimmung.

**Tabelle 5**

| **Anti-inflammatorische Wirkung (Angabe in %-rel.)** | | | |
|---|---|---|---|
| **Zuckerester** | **Konz. %w/v** | **Zellzahlen** | **Freigesetzte ROS** |
| Blindprobe + Stimulation | 0 | 100 | 100 |
| Fructosecaprat + Stimulation | 0,003 | 97 | 87 |
| | 0,01 | 60 | 5 |
| Fructosepalmitat + Stimulation | 0,001 | 97 | 81 |
| | 0,01 | 98 | 42 |
| Fructosestearat + Stimulation | 0,001 | 100 | 88 |
| | 0,01 | 155 | 26 |
| Glucosepalmitat + Stimulation | 0,001 | 102 | 99 |
| | 0,01 | 91 | 58 |

Die Ergebnisse zeigen, dass die Testsubstanzen einen starken inhibierenden Einfluss auf den Atmungsausbruch menschlicher Granulocyten besitzen, ohne die Granulocyten zu schädigen.

### E) Wirksamkeit gegen Proteasen (Illustratives Beispiel)

Während einer Inflammation, werden aus den polymorphonuclearen neutrophilen Granulocyten oder Makrophagen Hautproteasen, wie beispielsweise Collagenase freigesetzt. Ein ähnlicher Vorgang spielt sich gerade in der Haut älterer Menschen bei Einfluss von UV-Strahlen ab. Die Proteasen - wegen ihres Gehaltes an zentralen Zinkionen auch als Matrix-Metallo-Proteasen (MMP) bezeichnet - katalysieren wie schon erwähnt die Fragmentierung von Bindegewebsproteinen. Zur Untersuchung der Testsubstanzen auf Collagenaseinhibierung wurde bakterielle Collagenase (clostridium histolyticum) auf Gelatine als natürlichem Nährboden verwendet, welche mit Fluorochrom (FITC, Calbiochem) markiert war. Die Inkubationszeit betrug 60 min bei 20 °C, die Hydrolyse des Substrates wurde über die Fluoreszenz bei 393 nm (Anregung bei 328 nm) verfolgt. Die Ergebnisse sind in Tabelle 6 zusammengefasst. Angegeben ist die Collagenase-Inhibierung in %.

**Tabelle 6**

| **Collagenase-Inhibierung (Angabe in %-rel.)** | | |
|---|---|---|
| **Zuckerester** | **Konz. [w/v]** | **Collagenase-Inhibierung** |
| Blindprobe | 0 | 0 |
| Fructosepalmitat | 0,03 | 4 |
| | 0,1 | 41 |
| | 0,3 | 78 |
| Fructosestearat | 0,3 | 45 |
| Glucoselaurat | 0,1 | 28 |
| Trehalosecaprat | 0,3 | 52 |
| Trehaloselaurat | 0,1 | 21 |
| Trehalosepalmitat | 0,3 | 9 |

Die Ergebnisse zeigen, dass die Testsubstanzen in Abhängigkeit der Konzentration über eine signifikante Inhibierungswirkung verfügen.
Insofern können die untersuchten Gycosidfettsäureester erfolgreich eingesetzt werden gegen Hautalterung, denn die Collagenaseaktivität nimmt während des Alterungsprozesses deutlich zu.
Des Weiteren sind sie gegen Hautalterung anzuwenden, die speziell durch oxidativen Stress, UV-Strahlung oder Umweltgifte beschleunigt wird, denn es ist bekannt, dass gerade diese Faktoren die Freisetzung aktiver Collagenase bewirken.

### F) Inhibierung der Melaninsynthese in B16-Melanocyten

Melanin ist für die Pigmentierung von Haut und Haaren verantwortlich. Die Synthese dieses Pigmentes findet in speziellen Organellen, den sogenannten Melanosomen statt, welche in den Melanocyten der Basalschicht der menschlichen Epidermis zu finden sind. Die Biosynthese geht von der Aminosäure Tyrosin aus, die in Gegenwart von Tyrosinase zu DOPA (Dihydroxyphenylalanin) oxidiert wird, welches dann seinerseits zu Melanin polymerisiert. Zum Nachweis der Inhibierung der Melanininhibierung wurden Melanocyten (B16 Zelllinie) in einem Standardmedium inoculiert. Nach einer Inkubationszeit von 3 Tagen bei 37 °C und 5 Vol.-% CO₂ wurde das Nährmedium gegen eine Lösung gewechselt, welche die Testsubstanzen in unterschiedlichen Konzentrationen enthielt. Nach einer erneuten Inkubationszeit von 3 Tagen wurde der Gehalt an Zellproteinen (Bradford-Methode) und gebildetem Melanin über die optische Dichte des Homogenisates bei 475 nm bestimmt. Die Ergebnisse sind angegeben in %-rel gegenüber einem Blindwert und in Tabelle 7 zusammengefasst.

**Tabelle 7:**

| **Melaninsynthese in B16 Melanocyten [%-rel.]** | | | |
|---|---|---|---|
| **Zuckerester** | **Konz. w/v.** | **Zellproteine** | **Zellmelanin** |
| Blindwert | 0 | 100 | 100 |
| Fructosecaprat | 0,003 | 108 | 54 |
| | 0,006 | 80 | 30 |
| Fructosedicaprat | 0,003 | 105 | 88 |
| | 0,01 | 91 | 41 |
| Fructosestearat | 0,006 | 105 | 49 |
| | 0,01 | 76 | 21 |
| Fructosemonostearat | 0,001 | 104 | 85 |
| | 0,003 | 91 | 34 |
| Trehalosecaprat | 0,001 | 100 | 53 |
| | 0,003 | 75 | 23 |
| Trehalosestearat | 0,001 | 97 | 81 |
| | 0,003 | 93 | 41 |
| Saccharosestearat ¹⁾ | 0,001 | 89 | 75 |
| | 0,003 | 70 | 18 |

| | | | |
|---|---|---|---|
| 1) Ryoto S1670 | | | |

Die Ergebnisse zeigen, dass die Testsubstanzen die Melaninsynthese in den B16-Melanocyten signifikant inhibieren.

### G) Wachstumsinhibierung an Human-Keratinocyten (Illustratives Beispiel)

Eine gesteigerte Proliferation und Differenzierung von Keratinocyten der Haarmatrix führt zu einem verbesserten längeren Haarwachstum. Die nachfolgende Untersuchung wurde durchgeführt, um das Potential der Zuckerester zur Inhibierung des Haarwachstums zu bestimmen, das durch eine Reduzierung der Proliferation der menschlichen Keratinocytenkultur in vitro sichtbar wird.
Zu diesem Zweck wurden humane Keratinocyten in einem Standardzellkulturmedium mit foetalem Kälberserum (FCS) angezüchtet. Nach der Inkubation über einen Tag bei 37°C und CO₂= 5% wurde das Wachstumsmedium durch Standardmedium mit 10 ng/ml Epidermalem Wachstumsfaktor (EGF) und mit unterschiedlichen Konzentrationen von Zuckerestern, gelöst in Ethanol (1 Vol.%), ausgetauscht.
Nach einer dreitägigen Inkubation wurde die Anzahl der lebenden Zellen über die Bestimmung des zellulären Proteingehaltes nach Bradford [Bradford M.M. A rapid and sensitive method for the quantitation of microgram quantities of protein utilizing the principle of protein-dye binding. Anal. Biochem. (1977) vol 72, pp 248-254] ermittelt.
Die Ergebnisse der Tabelle 8 stellen die Werte von Dreifachbestimmungen an zwei Ansätzen dar, ausgedrückt in % gegen eine Kontrolle aus Zellkulturmedium ohne Zuckerester.

**Tabelle 8:**

| **Wachstumshemmung an Human-Keratinocyten (in % gegen Kontrolle)** | | |
|---|---|---|
| Zuckerester | Konz. % w/v | Zellproteingehalt in % |
| Kontrolle* | 0 | 100 |
| Kontrolle mit EGF * | 0 | 167 |
| Fructosecaprat mit EGF | 0,001 | 163 |
| | 0,003 | 130 |
| | 0,01 | 45 |
| Fructosepalmitat mit EGF | 0,001 | 166 |
| | 0,003 | 143 |
| | 0,01 | 105 |

| | | |
|---|---|---|
| * Kontrollexperimente wurden ausgeführt, indem 1 Vol.% Ethanol ohne Zuckerester eingesetzt wurde. Sie zeigten auch, dass Ethanol keinen signifikanten Effekt auf das Wachstumsverhalten der Keratinocyten hatte. | | |

Die untersuchten Zuckerester verringerten die Zellproliferation von Human-Keratinocyten, die mit EGF in vitro kultiviert wurden signifikant. Sie bewiesen damit ein hohes Potential zur Inhibierung des Haarwachstums.

### H) Inhibierung des Haarwachstums (Illustratives Beispiel)

Zur Bestimmung des haarwuchshemmenden Effektes der Zuckerester wurde eine Methode mit in vitro kultivierten humanen Haarfollikeln in einem Wachstumsmedium angewandt.
Es wurde das Wachstum der Haarfollikel in An- und Abwesenheit von Fructosecaprat untersucht. Dazu wurden die Haarfollikel über 7 Tage bei 37°C und CO₂= 5% inkubiert. Die Länge der kultivierten Haarfollikel wurde nach 3 und nach 7 Tagen der Kultivierung aufgenommen. In einem Vergleichsbeispiel enthielt das Wachstumsmedium zusätzlich 30 ng/ml IGF-1 (Insulin like growth factor), ein Cytokin, das Haarfollikelwachstum stimuliert.

Jeder der Versuche wurde mit 10 Haarfollikeln eines Spenders (insgesamt 5 Spender) durchgeführt.
Die Ergebnisse in Tabelle 9 stellen Durchschnittswerte der 50 Haarfollikel dar.

**TABLE 9**

| **Haarfollikelwachstum - Zunahme des Haarwachstums nach 3 und 7 Tagen Inkubation - bezogen auf die Anfangslänge (Tag 0)** | | | |
|---|---|---|---|
| Follikelbehandlung | Konz. %w/v | Tag 3/ Tag 0 | Tag 7/ Tag 0 |
| Kontrolle | 0 | +15% | +32% |
| Fructosecaprat | 0,01 | +12% | +21% |
| Kontrolle | 0 | +21% | +45% |
| Fructosecaprat + IGF | 0,01 | +15% | +25% |

Die Ergebnisse zeigen bei Abwesenheit von IGF eine deutliche Abnahme des Haarwachstums nach Behandlung mit den untersuchten Zuckerestern bereits in einer Konzentration von 0,01 Gew.%. Der Unterschied wird noch deutlicher bei Anwendung der IGF haltigen Lösung.

### I) Antibakterielle Wirksamkeit (Illustratives Beispiel)

Die antibakterielle Wirksamkeit der Testsubstanzen wurde mit Hilfe der Diffusionsmethode auf Agar-Platten bzw. der Agar-Verdünnungsmethode untersucht. Dabei wird zunächst ein rundes Filterpapier definierten Durchmessers mit der Testlösung getränkt und dieses dann auf die Oberfläche einer Agarplatte aufgebracht, welche zuvor mit den Testmikroorganismen inokuliert worden war. Anschließend wird nach definierten Zeiten die Größe der Inhibierungszonen bestimmt. Diese Technik erlaubt es insbesondere die MIC (Minimum Inhibitory Concentration) als die niedrigste Testsubstanzkonzentration zu bestimmen, mit der noch eine vollständige Inhibierung der Mikroorganismen erzielt werden kann.

*Agar-Diffusionsmethode.* Das Inokulum wurde unter Verwendung einer frischen Kultur in einer stationären Wachstumsphase (nach ca. 18-24 h) in einer BHI-Lösung (Brain-Heart-InfusionI) hergestellt. Die Bakteriensuspension wurde auf 0,5 MacFarland-Einheiten eingestellt, entsprechend 1,5 10⁸ Kolonienbildende Einheiten (cfu)/ml; anschließend wurde die Suspension mit einer Kochsalzlösung 1/100 verdünnt, um einen Wert von 1,5 10⁶ cfu/ml einzustellen. Anschließend wurde Mueller-Hinton Agar (*Staphylococcus epidermis, Staphylococcus aureus*) und Wilkins-Chalgrens Agar mit 5 Gew.-% Schafsblut (*Propionobakterium acnes*) 15 min bei 121 °C sterilisiert und dann in Petrischalen gegeben. Die Petrischalen wurden mit 2 bis 4 ml der Bakteriensuspensionen versetzt und bei Raumtemperatur getrocknet. Die Filterpapiere wurden mit 20 µl der Testsubstanzen getränkt und auf die Oberfläche der Agarplatten aufgebracht. Die inokulierten Petrischalen wurden 18 bis 24 bei 37 °C inkubiert (die Petrischalen mit Propionibacterium acnes unter anaeroben Bedingungen) und die antimikrobielle Wirksamkeit anschließend durch Bestimmung der Wachstumsinhibierungszone ermittelt. Die Ergebnisse sind in Tabelle 10 zusammengefasst. Angegeben sind ist jeweils der Durchmesser der Inhibierungsflächen in mm.

*Agar Verdünnungsmethode (MIC-Bestimmung)*. Wie oben beschrieben wurden verschiedene Agars vorbereitet, mit unterschiedlichen Konzentrationen an Testsubstanzen versetzt, homogenisiert und dann getrocknet. Anschließend erfolgte die Inokulation der Petrischalen mit jeweils 2 µl der Bakteriensuspensionen. Nach dem erneuten Trocknen wurden die Petrischalen bei 37 °C 18 bis 24 h inkubiert. In Tabelle 11 sind die MIC in mg/ml angegeben, d.h. die geringsten Konzentrationen, mit denen noch eine vollständige Inhibierung des Bakterienwachstums erreicht werden kann. Es handelt sich jeweils um den Mittelwert von Doppelbestimmungen.

**Tabelle 10:**

| **Inhibierungszonen [mm]** | | | |
|---|---|---|---|
| **Zuckerester** | **Staphylococcus aureus** | **Staphylococcus epidermidis** | **Propionibacterium acnes** |
| Fructosecaprat | 10 | 9 | 14 |
| Glucosecaprylat | 11 | 0 | 12 |
| Glucoselaurat | 14 | 16 | 10 |
| Trehalosecaprat | 10 | 0 | 10 |
| Trehalosepalmitat | 14 | 11 | 0 |
| Trehalosestearat | 12 | 11 | 0 |

**Tabelle 11:**

| **MIC [mg/ml]** | | | |
|---|---|---|---|
| **Zuckerester** | **Staphylococcus epidermidis** | **Staphylococcus aureus** | **Propionibacterium acnes** |
| Fructosecaprat | 1,25 | 1,25 | 0,625 |
| Trehalosestearat | 0,625 | >10 | 1,25 |

Die Ergebnisse zeigen, dass die Testsubstanzen über ausgezeichnete antimikrobielle Eigenschaften speziell gegenüber solchen Keimen verfügen, die in die Entstehung von Akne involviert sind.
In der nachfolgenden Tabelle finden sich eine Reihe von Formulierungsbeispielen.

**Tabelle 12**

| **Weiße Emulsionen mit Zuckerestern als Emulgatoren (Mengenangaben als Gew.-%)** | | | | | |
|---|---|---|---|---|---|
| **Phase** | **Inhaltsstoff** | **A1** | **A2** | **A3** | **A4** |
| Phase I | Cetearyl Alcohol | 3 | 3 | 3 | 3 |
| | Cetearyl Isononanoate | 15 | 15 | 15 | 15 |
| | Caprylic Capric Triglycerides | 6 | 6 | 6 | 6 |
| | Fructosepalmitat | 3 | - | - | - |
| | Trehalosepalmitat | - | 3 | - | - |
| | Trehalosestearat | - | - | 3 | - |
| | Sucrosestearat ¹⁾ | - | - | - | 3 |
| Phase 2 | Wasser | ad 100 | | | |
| | Elestab® 388 | 2,5 | 2,5 | 2,5 | 2,5 |
| | Keltrol® T | 0,2 | 0,2 | 0,2 | 0,2 |
| | NaOH 1N | 0,2 | 0,2 | 0,2 | - |
| Phase 3 | Citric acid (10 % b.w.) | - | 0,1 | 0,1 | - |

| | | | | | |
|---|---|---|---|---|---|
| 1) Sisterna SP50C | | | | | |

Zur Herstellung der Emulsionen wurde die Phase 1 bei 75 °C vorgelegt und die auf die gleiche Temperatur erhitzte Phase 2 unter starkem Rühren hinzugegeben. Nach Abkühlen auf Raumtemperatur wurde die Phase 3 eingerührt. Die Emulsionen wiesen in 5 Gew.-%iger Verdünnung einen pH-Wert von 6,2 bis 7,8 sowie eine Viskosität nach Brookfield von 120 bis 600 ps auf.

**Tabelle 13**

| **Weiße Emulsionen mit Zuckerestern als Co-Emulgatoren (Mengenangaben als Gew.-%)** | | | | | | | |
|---|---|---|---|---|---|---|---|
| **Phase** | **Inhaltsstoff** | **B1** | **B2** | **B3** | **B4** | **B5** | **B6** |
| Phase 1 | Cetearyl Alcohol (and) Ceteareth 20 | 5 | 5 | 5 | 5 | 5 | 5 |
| | Cetearyl Alcohol | 3 | 3 | 3 | 3 | 3 | 3 |
| | Cetearyl Isononanoate | 15 | 15 | 15 | 15 | 15 | 15 |
| | Caprylic Capric Triglycerides | 3 | 3 | 3 | 3 | 3 | 3 |
| | Fructosecaprat | 3 | - | - | - | - | - |
| | Glucosecaprylat | - | 3 | - | - | - | - |
| | Glucoselaurat | - | - | 3 | - | - | - |
| | Trehalosecaprat | - | - | - | 3 | - | - |
| | Trehaloselaurat | - | - | - | - | 3 | - |
| | Sucroselaurat ¹⁾ | - | - | - | - | - | 3 |
| Phase 2 | Wasser | ad 100 | | | | | |
| | Elestab® 388 | 2,5 | 2,5 | 2,5 | 2,5 | 2,5 | 2,5 |
| | Keltrol® T | 0,2 | 0,2 | 0,2 | 0,2 | 0,2 | 0,2 |
| | NaOH 1N | - | 0,2 | 0,2 | 0,1 | 0,1 | - |

| | | | | | | | |
|---|---|---|---|---|---|---|---|
| 1) Ryoto L595 | | | | | | | |

Zur Herstellung der Emulsionen wurde die Phase 1 bei 75 °C vorgelegt und die auf die gleiche Temperatur erhitzte Phase 2 unter starkem Rühren hinzugegeben. Die Emulsionen wiesen in 5 Gew.-%iger Verdünnung einen pH-Wert von 6,2 bis 7,2 sowie eine Viskosität nach Brookfield von 200 bis 600 ps auf.

**Tabelle 14**

| **Schäumende Zubereitungen (Mengenangaben als Gew.-%)** | | | | | | | |
|---|---|---|---|---|---|---|---|
| **Inhaltsstoff** | | **C1** | **C2** | **C3** | **C4** | **C5** | **C6** |
| Phase 1 | Fructosecaprat | 2,5 | - | - | - | - | - |
| | Glucosecaprylate | | 2,5 | - | - | - | - |
| | Glucoselaurat | - | - | 2,5 | - | - | - |
| | Trehalosecaprat | - | - | - | 2,5 | - | - |
| | Trehaloselaurat | - | - | - | - | 2,5 | - |
| | Sucroselaurat ¹⁾ | - | - | - | - | - | 6.25 |
| | Elestab® 388 | 2,5 | 2,5 | 2,5 | 2,5 | 2,5 | 2,5 |
| | Wasser | ad 100 | | | | | |
| Phase 2 | Sodium Laureth Sulfate | 30 | 30 | 30 | 30 | 30 | 30 |
| | Cocamidopropyl Betaine | 6 | 6 | 6 | 6 | 6 | 6 |
| | Sodium chloride | 0,5 | 0,5 | 0,5 | 0,5 | 0,5 | 0,5 |

| | | | | | | | |
|---|---|---|---|---|---|---|---|
| 1) Sisterna L 70C | | | | | | | |

Zur Herstellung der Zubereitungen wurde die Phase 1 bei 75 °C vorgelegt, zunächst das Sodium Laureth Sulfate eingerührt und dann nach Abkühlen auf Raumtemperatur die übrigen Bestandteile der Phase II hinzugegeben. Die Emulsionen wiesen in 5 Gew.-%iger Verdünnung einen pH-Wert von 6 bis 7 sowie eine Viskosität nach Brookfield von 200 bis 2500 cps auf.

## Patentansprüche

1. Nicht-therapeutische, kosmetische Verwendung von Zuckerestern als Wirkstoffe zur Inhibierung der Melaninsynthese in Haar- und Hautzellen, wobei man Zuckerester einsetzte die sich von Fettsäuren der Formel **(I)** ableiten,
**R¹CO-OH** **(I)**
in der R¹CO für einen linearen oder verzweigten, gesättigten oder ungesättigten Acyl oder Hydroxyacylrest mit 6 bis 22 Kohlenstoffatomen und 0 und/oder 1 bis 3 Doppelbindungen steht , wobei man Zuckerester einsetzt, die sich von Mono- oder Disachariden ableiten.

2. Verwendung nach Anspruch 1 **dadurch gekennzeichnet, dass** man Saccharide einsetzt, die ausgewählt sind aus der Gruppe, die gebildet wird von Glucose, Methylglucose, Mannose, Galactose, Rhamnose, Fucose, Fructose, Ribose, Desoxyribose, Arabinose, Xylose, Sucrose, Maltose, Isomaltose, Cellobiose, Melibiose, Gentobiose, Lactose und Trehalose.

3. Verwendung nach einem der Ansprüche 1 bis 2, **dadurch gekennzeichnet, dass** man Zuckerester einsetzt, die einen Veresterungsgrad von durchschnittlich 1 bis 6 aufweisen.

4. Verwendung nach einem der Ansprüche 1 bis 3, **dadurch gekennzeichnet, dass** man die Zuckerester in Mengen von 0,0001 bis 10 Gew.-% - bezogen auf die Mittel - einsetzt.

## Claims

1. The non-therapeutic, cosmetic use of sugar esters as active components for inhibiting the synthesis of melanin in skin and hair cells, wherein sugar esters derived from fatty acids corresponding to formula **(I)**:
**R¹CO-OH** **(I)**
in which R¹CO is a linear or branched, saturated or unsaturated acyl or hydroxyacyl radical containing 6 to 22 carbon atoms and 0 and/or 1 to 3 double bonds, are used, wherein sugar esters derived from mono- or disaccharides are used.

2. The use claimed in claim 1, **characterized in that** saccharides selected from the group consisting of glucose, methyl glucose, mannose, galactose, rhamnose, fucose, fructose, ribose, deoxyribose, arabinose, xylose, sucrose, maltose, isomaltose, cellobiose, melibiose, gentobiose, lactose and trehalose are used.

3. The use as claimed in one of claims 1 to 2, **characterized in that** sugar esters with an average degree of esterification of 1 to 6 are used.

4. The use as claimed in one of claims 1 to 3, **characterized in that** the sugar esters are used in quantities of 0.0001 to 10% by weight, based on the preparation.

## Revendications

1. Utilisation non thérapeutique, cosmétique d'esters de sucres en ta nt que substances actives pour l'inhibition de la synthèse de la mélanine dans les cellules des cheveux et de la peau, dans laquelle on utilise des esters de sucres qui sont dérivés d'acides gras de formule (I),
R¹CO-OH (I)
dans laquelle R¹CO représente un radical acyle ou hydroxyacyle linéaire ou ramifié, saturé ou insaturé comprenant 6 à 22 atomes de carbone et 0 et/ou 1 à 3 doubles liaisons, en utilisant des esters de sucres qui sont dérivés de monosaccharides ou de disaccharides.

2. Utilisation selon la revendication 1, **caractérisée en ce qu'**on utilise des saccharides qui sont choisis dans le groupe formé par le glucose, le méthylglucose, le mannose, le galactose, le rhamnose, le fucose, le fructose, le ribose, le désoxyribose, l'arabinose, le xylose, le sucrose, le maltose, l'isomaltose, le cellobiose, le mélibiose, le gentobiose, le lactose et le tréhalose.

3. Utilisation selon l'une quelconque des revendications 1 à 2, **caractérisée en ce qu'**on utilise des esters de sucre qui présentent un degré d'estérification de 1 à 6 en moyenne.

4. Utilisation selon l'une quelconque des revendications 1 à 3, **caractérisée en ce qu'**on utilise les esters de sucre en des quantités de 0,0001 à 10% en poids par rapport aux préparations.
